# EUROPÄISCHE PATENTANMELDUNG

(11) **EP 0 657 533 A1**
(43) Veröffentlichungstag der Anmeldung: **14.06.1995**
(21) Anmeldenummer: 94116611.8
(22) Anmeldetag: 21.10.1994
(51) Int. Cl.: C12N 15/06, C12N 5/20, A61K 39/395, C07K 16/28, C07K 16/30

(54) **Monoklonale Antikörper mit hoher Zytotoxizität gegen humanes CD16-Antigen, sowie bispezifische monoklonale Antikörper unter Verwendung derartiger monoklonaler Antikörper und des CD-30-HRS-3-Antikorpers**

(30) Priorität: 30.10.1993 DE 4337197
(71) Anmelder: BIOTEST PHARMA GMBH, D-63303 Dreieich (DE)
(72) Erfinder: Pfreundschuh, Michael, Prof. Dr. med., D-66424 Homburg / Saar (DE)
(74) Vertreter: Beil, Hans Chr., Dr.

(57) **Zusammenfassung**

Die vorliegende Erfindung betrifft ein Verfahren zur selektiven Herstellung von Hybridomazellinien, die einen murinen monoklonalen Antikörper (MAK) der Klasse IgG1 mit hoher NK-Zell-abhängiger Zytotoxizität induzierender Kapazität gegen humanes CD16-Antigen produzieren, indem man die Hybridomazellen im Selektionsmedium mit unstimulierten menschlichen NK (natural Killer-)Zellen kokultiviert.

Die Erfindung betrifft ferner eine auf diese Weise erhältliche Zellinie A9 der DSM-Hinterlegungsnummer ACC 2148 und einen daraus erhältlichen MAK sowie ein Verfahren zur Herstellung bispezifischer MAK's durch Fusionierung einer anti-CD30-Zellinie HRS-3 mit Hybridoma-Zellinien, die nach dem obengenannten Selektionsverfahren, insbesondere mit der A9-Linie der DSM-Hinterlegungsnummer ACC 2148 erhalten wird, wobei insbesondere der bispezifische MAK der HRS-3/A9-Zellinie mit der DSM-Hinterlegungsnummer ACC 2142 gewonnen wird.

Diese bispezifischen MAK's eignen sich zur Therapie bzw. zur Rückbildung etablierter humaner Tumore, insbesondere von humanen Hodgkin-Tumoren.

## Beschreibung

Die vorliegende Erfindung betrifft ein Verfahren zur selektiven Herstellung von Hybridomazellinien, die einen murinen monoklonalen Antikörper (MAK) der Klasse IgG1 mit hoher NK-Zell-abhängiger Zytotoxizität induzierender Kapazität gegen humanes CD16-Antigen produzieren, indem man die Hybridomazellen im Selektionsmedium mit unstimulierten menschlichen NK (natural Killer-)Zellen kokultiviert.

Die Erfindung betrifft ferner eine auf diese Weise erhältliche Zellinie A9 der DSM-Hinterlegungsnummer ACC 2148 und einen daraus erhältlichen MAK sowie ein Verfahren zur Herstellung bispezifischer MAK's durch Fusionierung einer anti-CD30-Zellinie HRS-3 mit Hybridoma-Zellinien, die nach dem obengenannten Selektionsverfahren, insbesondere mit der A9-Linie der DSM-Hinterlegungsnummer ACC 2148 erhalten wird, wobei insbesondere der bispezifische MAK der HRS-3/A9-Zellinie mit der DSM-Hinterlegungsnummer ACC 2142 gewonnen wird.

Diese bispezifischen MAK's eignen sich zur Therapie bzw. zur Rückbildung etablierter humaner Tumore, insbesondere von humanen Hodgkin-Tumoren.

Die Anwendung von bifunktionellen monoklonalen Antikörpern (Bi-MAK) zur Behandlung von Tumoren ist von verschiedenen Arbeitsgruppen publiziert worden. Basis aller Arbeiten ist die Idee, die körpereigene Toleranz gegenüber dem Tumor durch direktes Targeting der Tumorzellen mit zytotoxischen Effektorzellen zu durchbrechen. Der bisherige Einsatz bispezifischer monoklonaler Antikörper als Immunmodulatoren bestand hauptsächlich darin, die zytolytische Aktivität von T-Zellen über den CD3/T-Zell-Rezeptor-Komplex (CD3/TCR-Komplex) gegen Tumorzellen zu richten [Stearz UD, Bevan, M J: Proc. Natl. Acad. Sci. USA 83: 1453-1457 (1986); Perez et al., Nature 316: 354 (1985)]. Allerdings hatte dies den Nachteil, daß für eine effiziente Tumorzellyse eine vorherige in vitro Stimulierung der T-Lymphozyten durch ein zusätzliches Aktivierungssignal erforderlich war [Pupa SM et al: Int.J. Cancer 42: 455 (1988); Garrido MA et al: J Immunol 144: 2891 (1990)].

Eine andere Subpopulation humaner Lymphozyten mit der Fähigkeit zur Lyse von Tumorzellen wird durch natürliche Killerzellen (NK-Zellen) repräsentiert. Diese stellen zellmorphologisch große granulierte Lymphozyten (large granular lymphocytes, LGL) dar und sind immunphänotypisch durch das Fehlen des CD3/TCR-Komplex sowie die Expression von CD2-, CD56- und CD16-Antigenen gekennzeichnet [Anderson P et al: Nature 341: 159-162 (1989); Herberman RB, Ortaldo, JR: Science 214: 24 (1981); Lanier LL et al: J Immunol 136: 4480 (1986)]. Im Gegensatz zu T-Zellen zeigen NK-Zellen direkte zytolytische Aktivität gegen eine Reihe von "NK-Zell-empfindlichen" Tumorzellen ohne vorherige Aktivierung. Jedoch ist die Mehrzahl der frischen und kultivierten Tumorzellen relativ resistent gegen die anti-Tumor-Wirkung von ruhenden peripheren NK-Zellen, die erst durch die Aktivierung mit Interleukin-2 (IL-2) in die Lage versetzt werden, gegen einen Teil dieser "NK-Zellresistenten" Tumorzellen zytolytische Aktivität zu entwickeln [Ferrini S et al: J Immunnol 138: 1297 (1987). Phillips JH, Lanier LL: J Exp Med 164: 814 (1986)]. Das CD16-Antigen ist ein niedrig affiner Rezeptor für den Fc-Teil komplexierter Immunglobuline (= Fc-gamma-III-Rezeptor) mit einem Molekulargewicht von 50-70 kDa, von dem verschiedene cDNA-Klone isoliert und sequenziert wurden. Zahlreiche funktionelle Untersuchungen des Fc-gamma-III-Rezeptors demonstrierten seine Bedeutung als die eines effektiven Aktivierungsantigens von NK-Zellen. Zugleich werden biologische Funktionen von NK-Zellen wie die ADCC über den Fc-gamma-III-Rezeptor vermittelt, deren Effektivität bei Verwendung monoklonaler Antikörper allerdings subklassenabhängig ist. Aufgrund der funktionellen Bedeutung des CD16-Antigens für die Aktivierung von NK-Zellen erschien daher eine selektive Induktion der Tumorzellyse via CD16 durch bispezifische Antikörper besonders vielversprechend zu sein. In der Tat war es möglich, mit Heterokonjugaten aus anti-CD16-Antikörpern und anti-DNP- (Dinitrophenyl-phosphat) bzw. Tumorzell-assoziierten-Antikörpern eine selektive Lyse von DNP-gekoppelten Hühnererythrozyten [Karpovsky B et al: J Exp Med 160: 1686 (1984)] und Tumorzellen einer in vitro etablierten humanen Kolonkarzinomzellinie [Titus JA et al: J Immunol 139:3153 (1987)] bzw. murinen T-Zellinie [Garrido MA et al: J Immunol 144: 2891 (1990)] durch unstimulierte und IL-2-aktivierte NK-Zellen zu induzieren. Titus JA et al [J Immunol 139: 153] beschrieben einen chemisch cross-linked Bi-MAK gegen CD16 und ein Melanomantigen, der in vivo im Nacktmausmodell effektiv das Tumorwachstum hemmte. Bi-MAK's gegen CD16 und Tumorantigene, die über Hybrid-Hybridom-Technologie (Tetradom-Technologie) gewonnen wurden, beschreiben die Arbeiten von Ferrini et al. [Int J Cancer Suppl 7: 15 (1992)] und Ring, D. [Cetus, PCT/US91/06949]. Diese Tetradome produzieren Bi-MAK's, die CD16-Antigen und Epidermal Growth Factor Receptor bzw. das Multi-Drug-Resistance-Antigen verknüpfen. Ebenso beschreiben Fanger et al. [Medarex, PCT/US90/05981] Bi-MAK gegen CD16 und tumorassoziierte Antigene.

Mit den beschriebenen bispezifischen MAK's ist somit zwar das Hemmen des Wachstums von Tumoren möglich, nicht jedoch eine vollkommene Rückbildung etablierter menschlicher Tumore.

Aufgabe vorliegender Erfindung ist es daher, CD16-MAK's zu entwickeln, welche selbst und in Verknüpfung mit bekannten MAK's als bispezifische Produkte die Fähigkeit aufweisen, eine maximale NK-Zytotoxizität zu induzieren, wodurch nicht nur das Wachstum menschlicher Tumore gehemmt wird, sondern vielmehr eine vollkommene Remission etablierter menschlicher Tumore erfolgt.

Diese Aufgabe wird erfindungsgemäß dadurch gelöst, daß man Hybridoma-Zellinien bereitstellt, die einen murinen MAK der Klasse IgG1 gegen humanes CD16-Antigen produzieren und eine hohe Aktivität bezüglich der Induktion NK-Zell-abhängiger Zytotoxizität aufweisen, indem man im Selektionsmedium die nach einer an sich bekannten Methode (Köhler/Milstein) gewonnenen Hybridoma-Zellinien mit unstimulierten menschlichen NK-Zellen, insbesondere Granulozyten, kokultiviert und die Zellen mit der höchsten Absterberate auswählt.

Überraschenderweise zeigte sich dabei, daß die Hybridomazellen, die CD-16-Antikörper mit besonders hoher Zytotoxizität-induzierender Aktivität bilden, die höchste Absterberate aufweisen, wenn sie mit ruhenden NK-Zellen kokultiviert werden.

Erfindungsgemäß erfolgt die Bestimmung der höchsten Absterberate durch Vergleich mit einem bekannten anti-CD16-MAK, wie z.B. VEB 13.

Auf diese Weise wird insbesondere die Zellinie A9 der DSM-Hinterlegungsnummer ACC 2148 erhalten, welche MAK's mit den obengenannten Eigenschaften aufweist.

Diese spezifisch auf die erfindungsgemäße Weise selektionierte Linie bzw. deren MAK's binden, wie Inhibierungsexperimente zeigen, an Epitope auf dem CD16-Antigen, welche entfernt sind von dem Bindungsepitop bekannter anti-CD16-MAK's, wie z.B. 3G8 (IgG1-Kappa, Dianova, Hamburg, DE), VEP 16 (IgM-kappa, Bering-Werke, Marburg, DE) oder Leu 11b (kommerziell erhältlich), wobei der von der Linie A9 abgeleitete MAK an eine weit entfernte und der von einer Linie C10 erhaltene MAK an eine dem von 3G8 bekannten Epitop etwas näher gelegene Bindungsstelle angreift. Darüberhinaus bindet der erfindungsgemäß hergestellte MAK A9 nur sehr schwach an lösliches rekombinantes humanes CD16, während die obengenannten bekannten Antikörper hier stärker binden. Dies zeigt, daß A9 andere Epitope erkennt als die bekannten anti-CD16-MAK's.

Der von A9 abgeleitete MAK ist ein Maus-IgG1-lambda-Subtyp. Die von der erfindungsgemäß hergestellten Zellinie A9 abgeleiteten MAK's sind in der Lage, bei Lymphozyten eine zytologische Aktivität gegen die den entsprechenden Antikörper produzierenden Hybridomazellen zu induzieren, wobei eine etwa 60%ige spezifische Lyse erfolgt, was mit den bisher bekannten MAK's nicht erzielt wurde.

Somit können diese Hybridomazellen, insbesondere die von der Linie A9 abgeleiteten Zellen, in an sich bekannter Weise mit HRS-3-Hydridomazellen zu Tetradomen (Hybrid-Hybridomen) fusioniert werden, aus welchen bispezifische MAK's, insbesondere aus der Linie HRS-3/A9 mit der DSM-Hinterlegungsnummer ACC 2142, erhalten werden, welche wirksam sind bei der Rückbildung menschlicher Tumore, insbesondere bei der Rückbildung etablierter Hodgkin-Tumore, wobei der Bi-MAK einerseits besser wirkt als die Einzelkomponenten A9 bzw. HRS-3 und andererseits besser wirkt als bereits bekannte obengenannte Bi-MAK's, mit welchen nur eine Hemmung des Tumorwachstums, nicht jedoch eine vollständige Rückbildung erzielt werden kann.

Die Hybridomazellen und die davon abgeleiteten MAK's bzw. die Bi-MAK's können auf folgende Weise hergestellt werden:

Immunisierte BALB/c-Maus-Milzzellen werden mit Maus-Myeloma-Zellen in bekannter Weise fusioniert. Die erhaltene Zellsuspension wird sodann im Selektionsmedium in Gegenwart humaner unstimulierter NK-Zellen, insbesondere Granuzlozyten, und gegebenenfalls peripheren Blutlymphozyten (PBL) kokultiviert. Durch Vergleich der anti-CD16-Aktivität mit bekannten CD16-Antikörpern, wie z.B. VEP 13, werden diejenigen Hybridomazellen selektioniert, welche die höchste Absterberate aufweisen. Nach diesem Prinzip der reversen Zytotoxiztät wird insbesondere die Linie A9 der DSM-Hinterlegungsnummer ACC 2148 erhalten.

Diese wird auf bekannte Weise durch Injektion in BALB/c-Mäuse vermehrt und durch chromatographische Reinigung aus der Ascitesflüssigkeit erhalten.

Zur Herstellung der Bi-MAK's, insbesondere des Bi-MAK's HRS-3/A9 mit zwei Leichtketten unterschiedlicher Klassen (lambda und kappa), werden HGPRT-negative HRS-3-Hybridomazellen (IgG1, kappa, Subtyp, wirksam gegen Hodgkin- und Reed-Sternberg-zell-assoziiertes CD30 Antigen, beschrieben von Engert et al, 1990), mit beispielsweise iodoacetamidbehandelten A9-Hybridomazellen fusioniert. Nach Prüfung auf bispezifische Reaktivität, z.B. durch Nachweis von Antikörpern mit unterschiedlichem Leichtkettengehalt durch indirekte Immunfluoreszenz, wird der gemischte Tetradomzellklon mit stärkster Simultanexpression beider Leichtketten erhalten und nachfolgend chromatographisch gereinigt.

In vivo Experimente mit dem Bi-MAK HRS-3/A9 zur Therapie menschlicher Hodgkin-Tumore in SCID-Mäusen zeigen, daß bei einer Vielzahl der getesteten Tiere eine komplette Remission der Tumore erfolgt.

Der erfindungsgemäß hergestellte Bi-MAK HRS-3/A9 kann nach bekannten Verfahren formuliert werden, z.B. als Lösung oder Lyophilisat, und dient zur parenteralen Anwendung an Menschen, wobei dem Fachmann bekannte Hilfs- und Zusatzstoffe vorhanden sein können.

Die Erfindung wird anhand der nachfolgenden Beispiele näher erläutert.

### I. Herstellung der CD16-MAK A9 und C10

### Beispiel 1:

### Immunisierung, Fusion und Klonierung der monoklonalen Antikörper A9 und C10

Zur Immunisierung wurden 3-4 Monate alte weibliche BALB/c-Mäuse verwendet. 3-5 x 107 frisch isolierte Granulozyten in 0,5 ml PBS wurden den Tieren in zweiwöchigen Abständen intraperitoneal injiziert. Die erste Injektion erfolgte als 1:1 Emulsion in komplettem Freund'schen Adjuvans, die zweite und dritte Injektion als 1:1 Emulsionen in inkomplettem Freund'schen Adjuvans. Die weiteren Immunisierungsschritte wurden ohne Adjuvans durchgeführt. Nach zehn Wochen bekamen die Tiere an den vier der geplanten Fusion vorangehenden Tagen jeweils eine Boost-Injektion von 5 x 107 Granulozyten.

Die immunisierten Milzzellen wurden mit AG8-653 Maus-Myeloma-Zellen im Verhältnis 2:1 in Gegenwart von Polyethylenglykol fusioniert. Die fusionierte Zellsuspension wurde einmal gewaschen und im Selektionsmedium (RPMI 1640, 10% fötales Kälberserum), 4 mM Glutamin, HAT: Hypoxanthin 13,6 mg/l; Aminopterin 0.17 mg/l und 10 µg/ml Insulin) kultiviert. Vierzehn Tage nach der Fusion wurden Überstände von Kulturen, die Hybridomawachstum zeigten, auf anti-CD16-Produktion getestet. Dazu wurden periphere Blutlymphozyten (PBL) und Granulozyten mit 10 µl Testüberstand oder Kontroll-Antikörper inkubiert. Als Kontroll-MAK wurde der kommerziell erhältliche CD16-Antikörper VEP13 eingesetzt. Der gebundene MAK wurde mittels eines FITC-markierten anti-Maus-Antikörpers im Zytofluorometer nachgewiesen. Von 1021 getesteten Überständen zeigten 4 ein CD16-spezifisches Bindungsmuster.

Nach 4 Klonierungsschritten unter Benutzung der "limiting dilution" Methode (Aussaatdichte 0,3 Zellen pro Kultur) wurden diese 4 Klone isoliert. A9 ist ein Maus IgG1-MAK mit einer Lambda leichten Kette, C10 ein Maus IgG1-MAK mit einer Kappa leichten Kette. Beide weisen eine signifikante Bindung an NK-Zellen auf.

### Beispiel 2:

### In vivo-Produktion und Reinigung der MAK A9 und C10

Die anti-CD16-MAK's A9 und C10 wurden in großen Mengen in vivo durch intraperitoneale Injektion von A9- bzw. C10-Hybridomazellen in BABL/c-Mäusen produziert. Eine Woche vor der Hybridomazellinjektion wurden die Mäuse mit 1,0 ml Pristan intraperitoneal geprimed. 8 - 14 Tage nach der Hybridomazellinjektion konnte Ascitesflüssigkeit abgenommen werden.

Die MAK's A9 und C10 wurden anschließend durch Ammoniumsulfatfällung (45% Sättigung) aus der Ascitesflüssigkeit präzipitiert. Danach erfolgte die weitere Reinigung der Antikörper durch eine Anionenaustauschchromatographie. Diese wurde mit einer Mono-Q-^{(R)}-Säule (Pharmacia, Freiburg) und einer FPLC-Anlage (Pharmacia, Freiburg) durchgeführt.

### II. Vergleich der MAX A9 und C10 mit anderen ant-CD16 MAK's

### Beispiel 3:

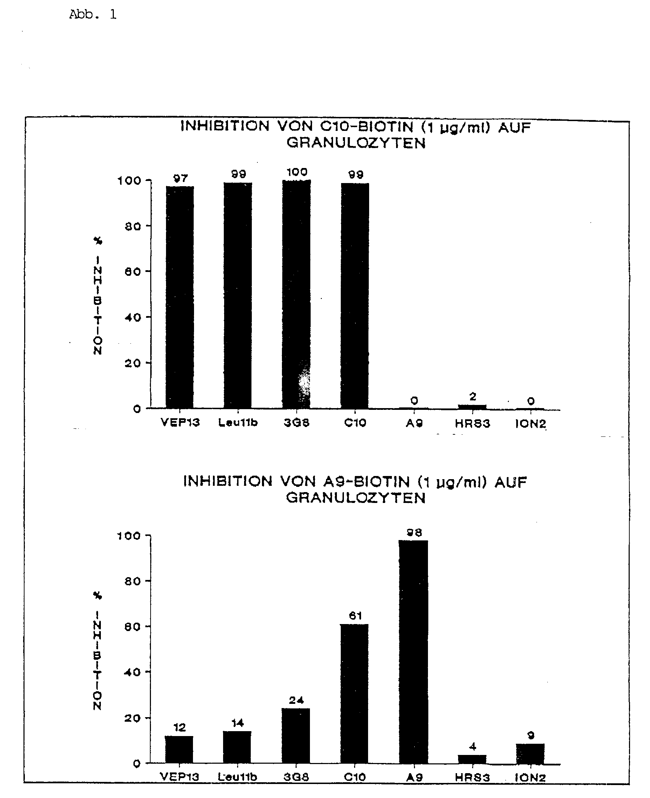

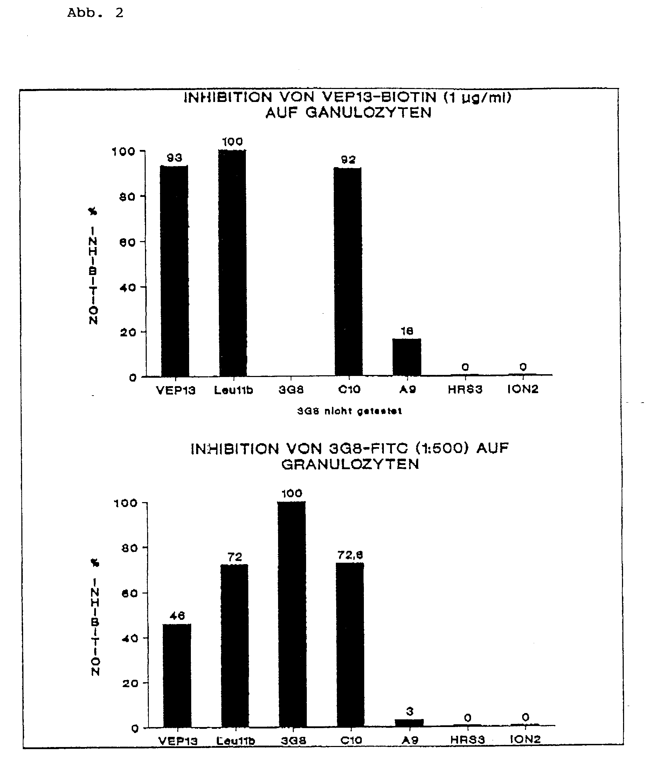

### Inhibitionsexperimente zur Epitop-Bestimmung (Abb. 1 und 2)

Nach Perussia et al. (J Immunol 133: 180 (1984)) existieren zumindest drei verschiedene, einander nicht überlappende Epitope auf dem CD16-Antigen. Um die von den erfindungsgemäß entwickelten CD16-MAK's A9 und C10 erkannten Epitope des CD16-Antigens genauer zu definieren, wurden Inhibitionsassays mit biotinylierten A9 und C10 MAK's und den kommerziell erhältlichen CD16-MAK's 3G8, VEP13 und Leu11b mittels direkter und indirekter Immunfluoreszenztests mit peripheren Lymphozyten und anschließender durchflußzytometrischer Auswertung durchgeführt. Dabei erkennen die Antikörper 3G8, VEP13 und Leu11b die gleichen oder doch sehr nahe beieinander liegende Epitope auf dem CD16-Antigen [Perussia et al.,(1984)). In den Abbildungen 1 und 2 sind repräsentative Ergebnisse der Inhibierungstests zusammengefaßt. Als markierte Antikörper dienten jeweils die biotinkonjugierten Antikörper A9, C10, VEP13 und der FITC-konjugierte Antikörper 3G8. Danach wurde C10-Biotin vollständig von 3G8, VEP13 und Leu11b inhibiert, und umgekehrt inhibierte C10 die anti-CD16-Antikörper VEP13-Biotin vollständig und 3G8-FITC partiell. Die Inhibition von VEP13-Biotin durch C10 erfolgte dosisabhängig, und zwar sowohl durch den kompletten Antikörper als auch durch C10-F(ab')2-Fragmente. A9-Biotin hingegen wurde von 3G8 und C10 nur partiell und von Leu11b und VEP13 überhaupt nicht inhibiert. Dabei inhibierte C10 die Bindung von A9-Biotin in weit größerem Ausmaß als 3G8. A9 selbst inhibierte keinen der übrigen markierten Antikörper. Im übrigen zeigten die getesteten anti-CD16-Antikörper untereinander eine ähnliche gegenseitige Inhibition wie von Perussia et al. (1984) beschrieben. Diese Ergebnisse sprechen dafür, daß A9 und C10 andere Epitope auf dem CD16-Antigen binden, und zwar A9 ein Epitop in weiter Entfernung von den von den anderen MAK's erkannten Epitopen, während das von C10 erkannte Epitop näher an dem von 3G8 erkannten Epitop liegt.

### Beispiel 4:

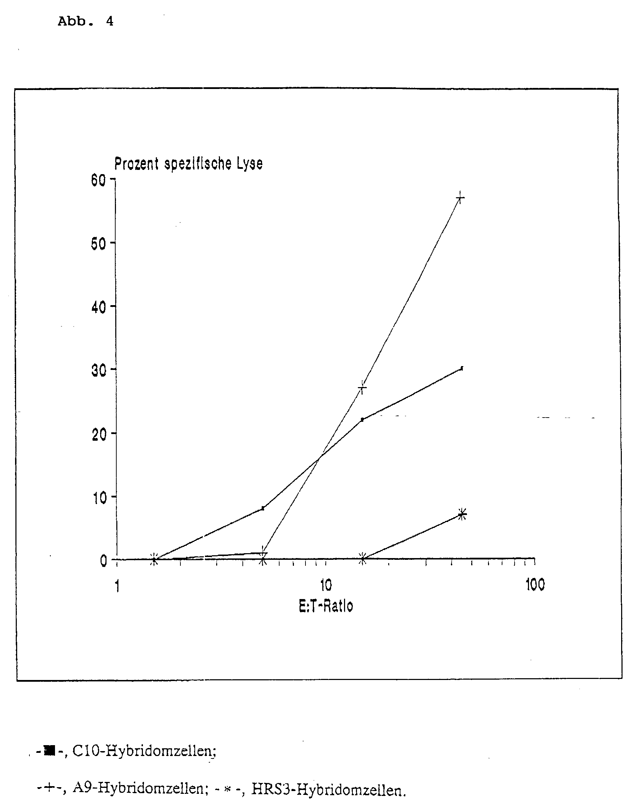

### Bindung der MAX A9 und C10 an lösliches rekombinantes humanes CD16 Antigen (Abb. 4)

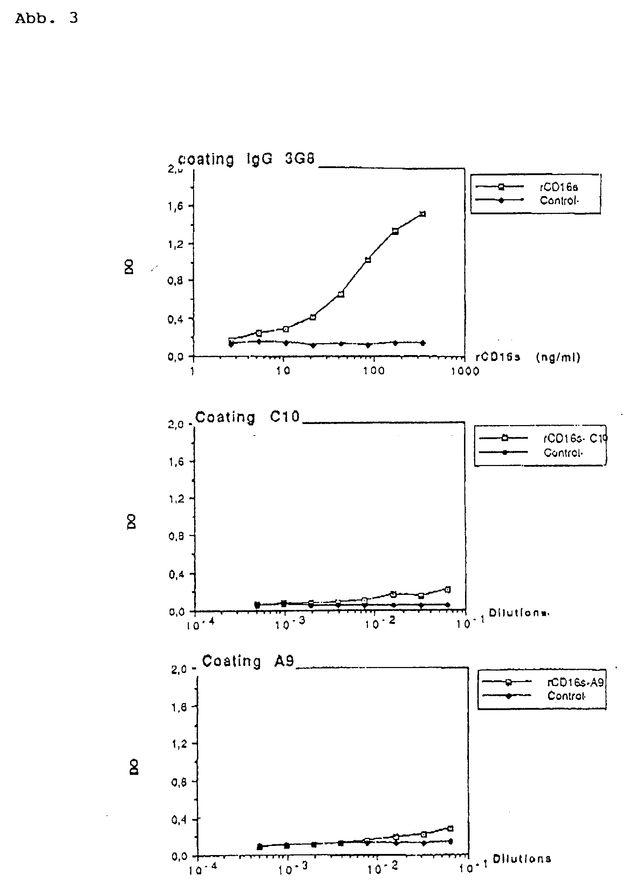

In einem Sandwich-ELISA, deren Ergebnisse in Abb. 3 dargestellt sind, mit löslichem rekombinantem humanem CD16 zeigte sich, daß sowohl A9 als auch C10 im Gegensatz zu den anderen obengenannten CD16-MAK nur sehr schwach an lösliches rekombinantes humanes CD16-Antigen binden. Dies bedeutet, daß sowohl A9 als auch C10 ein anderes Bindungsverhalten an lösliches CD16-Antigen zeigen als die übrigen Antikörper. Da beide Antikörper aber eine hohe Affinität gegen zellgebundenes CD16 zeigen, läßt sich dies nicht durch Affinitätsunterschiede erklären; vielmehr sprechen auch die ELISA-Ergebnisse dafür, daß die von A9 und C10 erkannten Epitope auf dem CD16-Antigen von den Epitopen, die von anderen CD16-MAK's erkannt werden, unterschiedlich sind.

### III. Biologische Aktivität der monoklonalen Antikörper A9 und C10

### Beispiel 5:

### Induktion von NK-Zell-abhängiger Zytotoxizität gegen CD16-MAK-produzierende Hybridomazellen (Abb. 4).

Hierzu wurden je 5 x 10³ ⁵¹Cr-markierte Hybridomzellen und mononukleäre Zellen in je 100 µl Medium im Verhältnis 45:1, 15:1, 5:1, 1,5:1 über 12 Stunden kokultiviert und aus der ⁵¹Cr-Freisetzung die spezifische Lyse bestimmt. Die Ergebnisse dieses Zytotoxizitätstests sind in Abb. 4 dargestellt. Es zeigte sich, daß die neu entwickelten Antikörper A9 und C10 im Gegensatz zu dem als Kontrolle eingesetzten Antikörper HRS-3 beide in der Lage waren, bei Lymphozyten zytolytische Aktivität gegen die den entsprechenden Antikörper produzierenden Hybridomzellen zu induzieren. Die durch A9 maximal induzierte Hybridomzell-Lyse betrug ca. 60%, spezifische Lyse, diejenige, die durch C10 induziert wurde, betrug jedoch nur ca. 30% spezifische Lyse.

### IV. Bispezifische monoklonale Antikörper

### Beispiel 6:

### Herstellung eines Bi-MAK aus dem MAX A9 und einem gegen ein menschliches tumorassoziiertes Antigen gerichteten zweiten MAK, HRS-3

Zur Herstellung von Bi-MAK's zur Induktion NK-Zell-vermittelter Zytotoxizität gegen menschliche Tumorzellen wurden HGPRT-negative HRS3-Hybridomzellen IgG1-kappa mit iodoacetamidbehandelten A9-Hybridomzellen fusioniert. Insgesamt wuchsen 113 Zellklone im Selektionsmedium (HAT-Medium) aus, von denen 17 Klone (=15%) bispezifische Reaktivität zeigten. Der Nachweis der Bispezifität erfolgte durch die Demonstration von Antikörpern mit unterschiedlichem Leichtkettengehalt durch indirekte Immunfluoreszenztest. Der Tetradomzellklon, der die stärkste simultane Expression beider Leichtketten zeigte, wurde mehrfach subkloniert, als Zellinie etabliert und der von dieser sezernierte bispezifische Antikörper HRS-3/A9 genannt. Die Trennung des bispezifischen Antikörpers von den ebenfalls durch die Tetradomazellen produzierten monospezifischen Ursprungsantikörpern HRS3 und A9 erfolgte aus HRS-3/A9-haltigem murinen Ascites über eine FPLC-Mono-Q^{(R)}-Anionenaustauschchromatographie. Die daraus resultierende, alle von den Tetradomzellen produzierten Antikörpervarianten enthaltende Gesamtimmunglobulinfraktion wurde anschließend durch eine FPLC-Mono-S^{(R)}-Kationenaustauschchromatographie weiter fraktioniert. Ein Vergleich der isoelektrischen Punkte der Antikörper HRS3 und A9 durch eine isoelektrische Fokussierung ergab, daß derjenige von A9 weiter im basischen Bereich lag als der von HRS3. Aus diesem Grund wurde für die chromatographische Auftrennung der Antikörper ein pH-Wert gewählt (=5,9), der knapp unterhalb des isoelektrischen Punkts von HRS3 lag, so daß die positive Ladungsdichte bei diesem Antikörper am niedrigsten war und er demzufolge vor HRS3/A9 und A9 eluiert werden mußte. Eluiert wurde mit einem diskontinuierlichen Salzgradienten mit 50 mmol/l MES von 0-250 mmol/l NaCl.

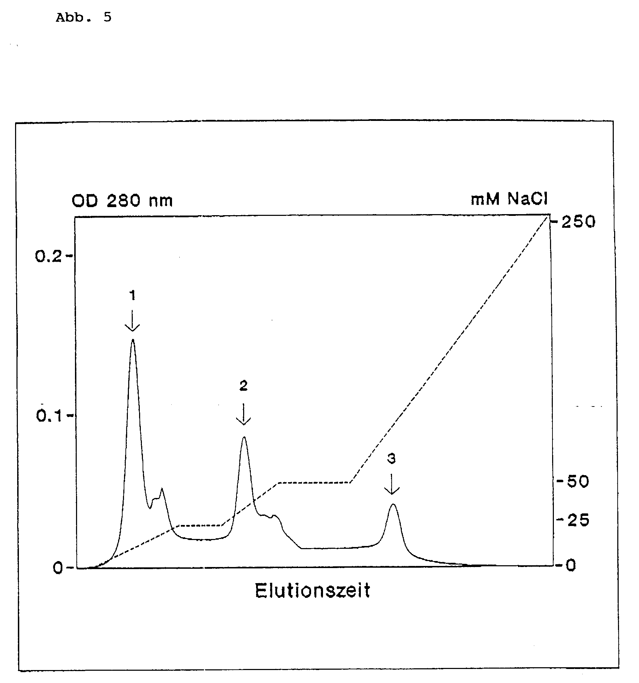

Die Abbildung 5 zeigt das resultierende Chromatogramm. Die Immunglobuline wurden in 3 Fraktionen (=3 Peaks) aufgetrennt, die anschließend auf den Gehalt von bispezifischen Antikörpern hin untersucht wurden. Die immunologische Untersuchung der 3 Fraktionen durch indirekte Immunfluoreszenz ergab, daß 2 Fraktionen (Peak 2) die bispezifische Aktivität enthielt.

### Beispiel 7:

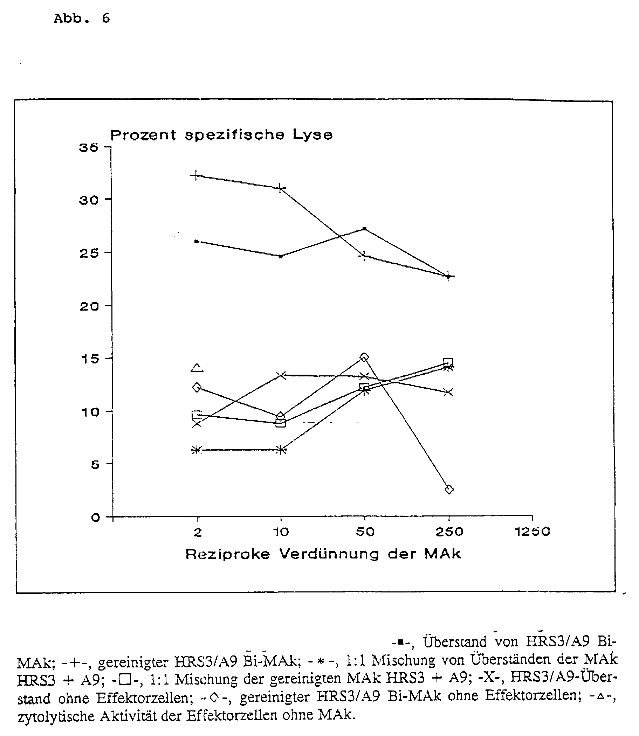

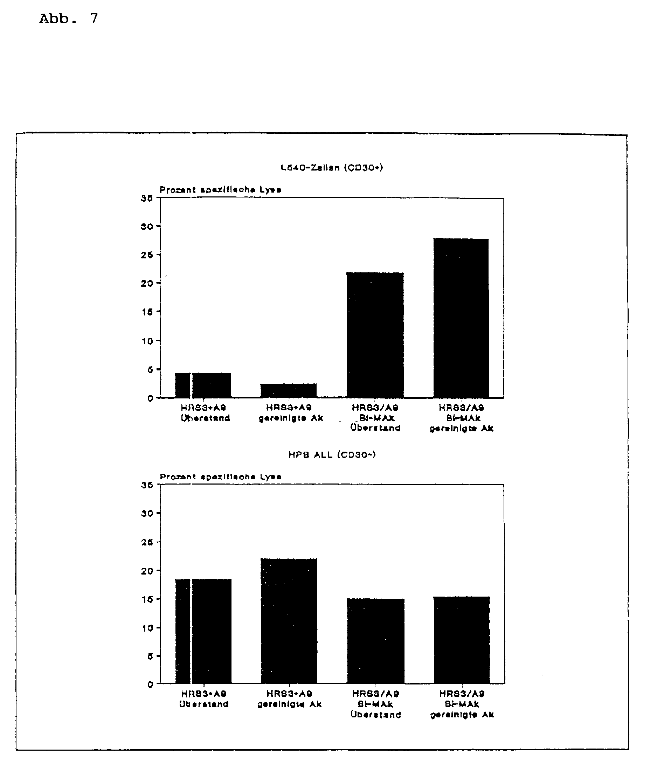

### Induktion einer NK-Zell-vermittelten Zytotoxizität gegen menschliche Tumorzellen in vitro (Abb. 6 und 7)

Über eine Percoll-Dichtegradientenzentrifugation mit NK-Zellen angereicherte Lymphozyten zeigten nur eine geringe zytolytische Aktivität gegen die CD30-Antigenpositive Hodgkin-Lymphom-Zellinie L540 (14% spezifische Lyse). Dagegen wurde bei Einsatz des bispezifischen Antikörpers HRS-3/A9 eine deutliche Erhöhung der zytolytischen Aktivität gegen L 540-Zellen beobachtet, und zwar sowohl durch bispezifischen Antikörper enthaltenden Zellkulturüberstand (bis zu 27% spezifische Lyse) als auch durch gereinigten bispezifischen Antikörper (bis zu 32% spezifische Lyse). Die zytolytische Aktivität der zur Kontrolle eingesetzten Ursprungsantikörper HRS-3 und A9 mit Effektorzellen und des bispezifischen Antikörpers ohne Effektorzellen lag lediglich zwischen 5-15%. Die Ergebnisse dieser Untersuchung sind in Abb. 6 dargestellt. Dabei wurde der Überstand 1:1 verdünnt, die höchste Konzentration der gereinigten Antikörper im Testansatz betrug 5 µg/ml. Beide wurden wie angegeben reziprok verdünnt. Das E:T-Verhältnis war 20:1.

Zugleich war diese Erhöhung der lytischen Aktivität spezifisch für die CD30- Zellinie L 540. Der bispezifische Antikörper HRS3/A9 war nur bei L 540-Zellen in der Lage, die zytolytische Aktivität von NK-Zellen zu verstärken, während keine Erhöhung der zytolytischen Aktivität gegen die CD30- Zellinie HPB-ALL, die ein gewisses Maß an spontaner "NK-Zell-Empfindlichkeit" zeigte, beobachtet wurde. Die Ergebnisse dieser Untersuchung sind in Abb. 7 dargestellt. Dabei wurde der Überstand 1:20 verdünnt, die Konzentrationen der gereinigten Bi-MAK betrugen 1 µg/ml. Das E:T-Verhältnis war 20:1.

Gleiche Ergebnisse wie mit angereicherten NK-Zellen wurden auch mit unstimulierten peripheren Blutlymphozyten (PBL) beobachtet. Die Induktion einer NK-Zell-vermittelten Zytotoxizität durch den Bi-MAK HRS-3/A9 bedarf also keiner Anreicherung von NK-Zellen. Dies ist insbesondere im Hinblick auf einen therapeutischen Einsatz dieses Antikörpers bei Patienten von Bedeutung.

### V. Präklinische Versuche mit dem Bi-MAK HRS-3/A9

### Beispiel 8:

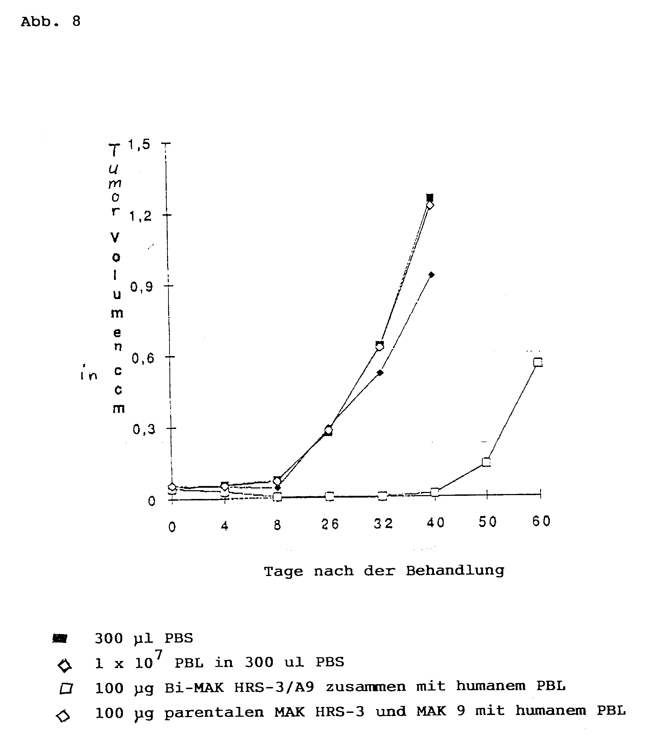

### Therapie von menschlichen Hodgkin-Tumoren in SCID-Mäusen mit Bi-MAK HRS-3/A9 und menschlichen PBL (Abb. 8)

Hodgkin-Tumoren wurden durch subkutane Injektion einer Suspension von 1.5 x 107 Zellen der menschlichen Hodgkin-Zellinie L540CY in die thorakale Wand von 4 - 6 Wochen alten weiblichen SCID-Mäusen etabliert. Nachdem die Tumoren einen Durchmesser von mindesten 5 mm erreicht hatten, erhielten jeweils 10 tumortragende Mäuse 100 µg Bi-MAK HRS-3/A9 oder einer gleichen Menge Kontrollantikörper und 1 x 107 menschlichen PBL intravenös. Bei 10/10 SCID-Mäusen mit etablierten menschlichen Hodgkin-Tumoren kam es zu einer vollständigen Rückbildung der Tumoren bis zum Tag 40, während alle anderen nicht behandelten Mäuse progredientes Tumorwachstum zeigten und bei Erreichen einer Tumorgröße von 10 mm Durchmesser getötet wurden. Bei 4 der spezifisch behandelten Mäuse kam es zu einem erneuten Tumorwachstum (Rezidiv) nach 60 Tagen (Abb.8). In einer weiteren Serie wurde bei weiteren 20/20 Mäusen eine komplette Remission durch Applikation von HRS-3/A9 und humanen PBLs erzielt. Dabei erhielten die Tiere am Tag 0 eine Injektion von
1) 300 µl PBS
2) 1 x 10⁷ humane periphere Blutlymphozyten PBL in 300 µl PBS
3) 100 µg Bi-MAK HRS-3/A9 mit humanem PBL
4) eine Mischung aus 100 µg parenteralen MAK HRS-3 und MAK A9 zusammen mit humanem PBL.

Das Wachstum der Tumore ist dargestellt als das durchschnittliche Tumorvolumen (ccm) aller tumortragender Tiere einer Behandlungsgruppe.

## Patentansprüche

1. Verfahren zur selektiven Herstellung von Hybridomazellinien, die einen murinen MAK der Klasse IgG1 mit hoher NK-Zell-abhängiger Zytotoxizität induzierender Kapazität gegen humanes CD16-Antigen produzieren, dadurch gekennzeichnet, daß man
a) CD16-MAK produzierende Hybridomazellen mit unstimulierten menschlichen NK-Zellen kokultiviert,
b) die Absterberate der Hybridomazellen bestimmt und nachfolgend
c) die Hybridomazellen mit höchster Absterberate selektioniert.

2. Verfahren gemäß Anspruch 1, dadurch gekennzeichnet, daß man die Kokultivierung gemäß Schritt a) in Gegenwart von Granulozyten durchführt und die Bestimmung der Absterberate gemäß Schritt b) durch Vergleich mit einem Kontroll-CD16-Antikörper erfolgt.

3. Verfahren gemäß Anspruch 1 oder 2, dadurch gekennzeichnest, daß man die Hybridomazellinie A9 der DSM-Hinterlegungsnummer ACC 2148 selektioniert.

4. Hybridomazellinie A9 der DSM-Hinterlegungsnummer ACC 2148, dadurch gekennzeichnet, daß sie einen monoklonalen Antikörper der Subklasse IgG1-lambda produziert, welcher eine hohe NK-Zell-abhängige Zytotoxizität induzierende Kapazität gegen humanes CD16-Antigen aufweist und an ein vom 3G8 erkanntes entferntes Epitop auf dem CD16-Antigen bindet.

5. Verfahren zur Herstellung bispezifischer MAK's, dadurch gekennzeichnet, daß man Hybridomazellen, hergestellt nach dem Verfahren gemäß einem der Ansprüche 1-3, in an sich bekannter Weise mit den Hybridomazellen HRS-3 gegen humanes CD30-Antigen zu einem Tetradom fusioniert und nachfolgend den erhaltenen bispezifischen MAK in an sich bekannter Weise isoliert und reinigt.

6. Verfahren gemäß Anspruch 6, dadurch gekennzeichnet, daß man die HRS-3-Zellen mit den Zellen erhältlich aus der Zellinie gemäß Anspruch 4 zu dem Tetradom HRS-3/A9 mit der DSM-Hinterlegungsnummer ACC 2142 fusioniert und den Bi-MAK gewinnt.

7. Verwendung eines bispezifischen MAK's, hergestellt nach dem Verfahren gemäß einem der Ansprüche 5 oder 6 zur Induktion der NK-Zell-vermittelten Zytotoxizität gegen menschliche Tumorzellen zur Rückbildung etablierter menschlicher Tumore.

8. Verwendung gemäß Anspruch 7 zur Rückbildung von menschlichen Hodgkin-Tumoren.
